# EUROPÄISCHE PATENTSCHRIFT

(11) **EP 3 692 191 B1**
(45) Veröffentlichungstag und Bekanntmachung des Hinweises auf die Patenterteilung: **03.04.2024**
(21) Anmeldenummer: 18779630.5
(22) Anmeldetag: 26.09.2018
(51) Int. Cl.: A61K 9/00, A61L 27/38, A61L 27/56, A61L 31/06, A61L 31/14

(54) **HOCHFLEXIBLE DEGRADIERBARE FASERN**
HIGHLY FLEXIBLE DEGRADABLE FIBERS
FIBRES DÉGRADABLES HAUTEMENT FLEXIBLES

(30) Priorität: 02.10.2017 DE 102017217539
(43) Veröffentlichungstag der Anmeldung: 12.08.2020
(73) Patentinhaber: Fraunhofer-Gesellschaft zur Förderung der angewandten Forschung e.V., 80686 München (DE)
(72) Erfinder: ROTHENBURGER-GLAUBITT, Miranda, 97276 Margetshöchheim (DE); CHRIST, Bastian, 97070 Würzburg (DE); GLAUBITT, Walther, 97276 Margetshöchheim (DE); PROBST, Jörn, 97573 Kürnach (DE)
(74) Vertreter: Pfenning, Meinig & Partner mbB
(86) Internationale Anmeldenummer: PCT/EP2018/076106
(87) Internationale Veröffentlichungsnummer: WO 2019/068533

(56) Entgegenhaltungen:
- WO-A1-2012/104320
- DE-A1-102007 061 873

## Beschreibung

Die vorliegende Erfindung betrifft Verfahren zur Herstellung von hochflexiblen, biodegradierbaren Fasern sowie die über das erfindungsgemäße Verfahren hergestellten Fasern und Fasergeflechte daraus. Außerdem betrifft die vorliegende Erfindung die Verwendung von biodegradierbaren Fasern oder Fasergeflechten in den Bereichen regenerative Therapien, Mikrobiologie, pharmazeutische Anwendungen, Kosmetikindustrie, Diagnostik, Lebensmittelbranche, Filtern, Faserverstärkung von Werkstoffen und Optik.

Mikro- und Nanofasern finden Anwendung in vielen Gesundheitsbereichen wie z.B. der Regenerativen Medizin, Therapie, Diagnostik, in der Pharmaindustrie sowie in der Lebensmittelbranche.

Eine bioabbaubare Faser, deren Abbauprodukte unter physiologischen Bedingungen nicht toxisch sind, ist beispielsweise geeignet, als Zellträgermaterial *in vitro* oder *in vivo* in der Regenerativen Medizin, Therapie und Diagnostik eingesetzt zu werden.

Aufgrund der Biokompatibilität von Siliziumdioxid sind SiOₓ-basierte Fasermaterialien hier von großem Interesse. Viele dieser Materialien finden Anwendung in Biologie, Medizin, der Lebensmittel- und Pharmaindustrie, sind jedoch meist nicht biologisch abbaubar. Die wenigen bekannten biologisch abbaubaren SiOₓ-Fasersysteme weisen eine geringe Flexibilität und eine hohe Neigung zur Sprödigkeit auf.

Ein in-vivo-Anwendungsbeispiel von Fasergeflechten ist als Zellträgerstruktur in einem Gewebedefekt. Bei der Verabreichung der Fasern in den Gewebedefekt verspröden diese aber sehr leicht und die Faserstruktur kann so die notwendige Stützfunktion zum Aufbau von neuem Gewebe nicht gewährleisten.

Die Sprödigkeit solcher Fasern bringt auch bei *in vitro-* Anwendungen praktische Probleme mit sich. Beispielsweise können solche Fasern nur sehr schwer zerstörungsfrei aus einer Wellplatte genommen werden.

Im Speziellen werden für Anwendungen in der Regenerativen Medizin biodegradierbare Materialien gesucht, die in einem Zeitraum von > 6 Monaten degradieren. Der Zeitraum über den die Degradierung der Faser erfolgt hängt stark von der Menge der verabreichten Faser und der Menge an Körperflüssigkeiten an der verabreichten Stelle ab. Deshalb kommen auch sehr große Zeiträume zur Degradierung, wie mehr als 3 Jahre, in Betracht. Bevorzugt ist es aber, dass die Faser in einem Zeitraum von 6 bis 18 Monaten und besonders bevorzugt von 10 bis 12 Monaten degradiert. Die meisten bisher entwickelten Fasermaterialien werden innerhalb weniger Tage oder Wochen abgebaut oder sind langzeitstabil (mehrere Jahre).

EP 2 152 785 A1 betrifft ein Polyethoxysiloxan(PES)-Material, das dadurch zu erhalten ist, dass (a) eine erste Hydrolyse-Kondensationsreaktion (HKR) von höchstens einem Rest X von einer oder von mehreren verschiedenen Si-Verbindungen der Formel I SiX4 (I), in der die Reste X gleich oder verschieden sind und Hydroxy, Wasserstoff oder Ethoxy (EtO) bedeuten, sauer katalysiert bei einem anfänglichen pH-Wert von 0 bis ≤ 7, in Gegenwart von Ethanol (EtOH) oder eines Ethanol-Wasser-Gemisches als Lösungsmittel, über einen Zeitraum von 1 bis 24 h bei einer Temperatur von 0°C bis 78°C durchgeführt wird, (b) eine zweite HKR des in Schritt (a) erhaltenen Materials bei gleichzeitigem Entfernen des Lösungsmittels durch sukzessives Eindampfen in einem gasdiffusionsdichten Behälter bei einem Druck von 100 bis 1013 mbar; vorzugsweise bei einem leichten Unterdruck von 300 mbar bis 800 mbar und einer Temperatur von 50 bis 78°C bis zu einer drastischen Viskositätserhöhung (bei einer Scherrate von 10 s⁻¹ bei 4°C) auf 0,5 bis 2 Pa s, bis zur Gewichtskonstanz und bis zur Bildung eines Cyclotetrasiloxans der allgemeinen Formel ((Si)(OH)_{0,75}(OEt)_{1,25} × 1/64 H₂O)₄ und der Molmasse von 4 * ca. 114 g = ca. 456 g, durchgeführt wird; (c) dieses PES-Material in einem geschlossenen Behälter in einem Zeitraum von wenigen Minuten bis wenigen Sekunden abgekühlt wird und (d) das aus (c) erhaltene PES-Material durch eine dritte HKR in ein rPES-Material überführt wird.

DE 10 2014 224 654 A1 beschreibt ein bioabbaubares Silikat-Hybridmaterial, enthaltend Silsesquioxane und einen organischen mehrarmigen Linker, wobei der organische mehrarmige Linker mindestens eine der Verbindungen I aufweist und kovalent an die Silsesquioxane gebunden ist.

Weitere relevante Informationen finden sich in den Dokumenten DE 10 2007 061873 und WO 2012/104320.

Die aus dem Stand der Technik bekannten bioabbaubaren Fasern degradieren innerhalb weniger Tage oder sind langzeitstabil (> 3 Jahre). Außerdem weisen diese Fasern nicht die benötigten mechanischen Eigenschaften, insbesondere keine ausreichend hohe mechanische Flexibilität, auf. Weiterhin sind die Biokompatibilität der Fasern und die Proliferation von humanen Fibroblasten auf diesen Fasern verbesserungswürdig.

Davon ausgehend bestand die Aufgabe vorliegender Erfindung darin ein Verfahren bereit zu stellen, welches es erlaubt Fasern herzustellen, die in einem Zeitraum von 6 bis 18 Monaten degradieren und die darüber hinaus sehr gute textil-mechanische Eigenschaften, insbesondere eine hohe mechanische Flexibilität, aufweisen. Weiterhin bestand die Aufgabe Fasern mit verbesserter Biokompatibilität, insbesondere bestimmt nach DIN ISO 10993-5, und einer erhöhten Proliferation von humanen Fibroblasten auf der Faser, bereitzustellen.

Diese Aufgabe wird mit dem Verfahren zur Herstellung biodegradierbarer Fasern gemäß Anspruch 1 gelöst, welches die folgenden Schritte enthält:
a) Bereitstellen von zumindest einer alkoholischen Lösung zumindest einer Silanverbindung ausgewählt aus der Gruppe bestehend aus Tetraalkoxysilanen, Trialkoxysilanen, Halogensilanen und Mischungen hiervon;
b) Bereitstellen einer wässrigen Lösung zumindest einer organischen Säure mit einem pK_{S}-Wert von < 2,0, wobei die organische Säure ausgewählt ist aus der Gruppe bestehend aus Sulfonsäuren, Schwefelsäureestern, Phosphonsäuren, Phosphorsäureestern und Mischungen hiervon;
c) Vermischen der in Schritt a) und b) bereitgestellten Lösungen und Durchmischen für 4 Stunden bis 1 Woche bei einer Temperatur von 20 bis 70°C;
d) Entfernen zumindest eines Teils des Alkohols aus der Mischung aus Schritt c) und anschließendes Abkühlen der konzentrierten Mischung auf eine Temperatur von 20 bis -25°C;
e) Aufbewahren der abgekühlten Mischung aus Schritt d) bis eine Viskosität von 10 bis 75 Pa·s erreicht ist;
f) Verspinnen der Mischung aus Schritt e) zu Endlosfasern; wobei während der Schritte c) bis e) eine Vernetzung der Silanverbindung erfolgt und zumindest ein Teil der organischen Säure über kovalente Bindungen in das entstehende Netzwerk eingebaut wird und/oder zur Vernetzung beiträgt.

Bevorzugte Ausführungsformen der erfindungsgemäßen biodegradierbaren Fasern werden in den abhängigen Ansprüchen 2 bis 10 angegeben.

Anspruch 11 betrifft eine nach dem erfindungsgemäßen Verfahren hergestellte Faser. Bevorzugte Ausführungsformen der erfindungsgemäßen biodegradierbaren Fasern werden in den Ansprüchen 12 und 13 angegeben.

Anspruch 14 betrifft Fasergeflechte und Filter aus den erfindungsgemäßen Fasern und Anspruch 15 betrifft Verwendungen der biodegradierbaren Fasern und Fasergeflechten.

### Begriffsdefinitionen

Eine "biodegradierbare" Faser im Sinne der vorliegenden Erfindung, meint eine Faser, die biokompatibel ist und unter physiologischen Bedingungen abgebaut wird.

Im Sinne der vorliegenden Erfindung meint der Begriff "Viskosität" die dynamische Viskosität, welche bevorzugt mit einem Rheometer der Fa. Anton Paar des Typs Physica MCR301 mit einem koaxialen Zylindermessaufsatz des Typs CC17/T200/SS und einer Scherrate von 10 s⁻¹ bestimmt wird.

Die "Vernetzung der Silanverbindung" und der kovalenten Einbau der organischen Säure in das Netzwerk wird in folgendem Schema anhand des Einbaus von Methansulfonsäure illustriert.

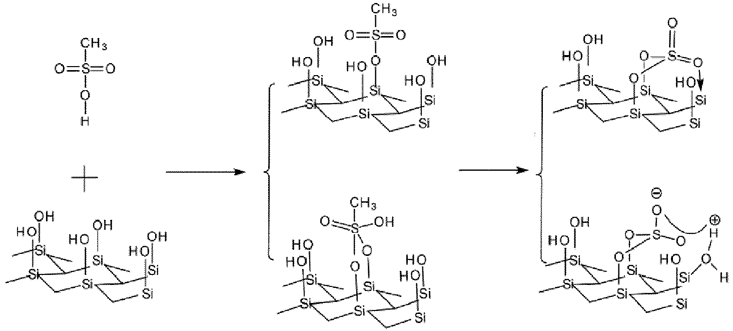

Unter dem Begriff "aufwickelbare Faser" wird eine Faser verstanden, die auf eine Rolle aufwickelbar ist, ohne dass die Faser dabei zerstört wird.

### Verfahren

Gemäß einer bevorzugten Ausführungsform der vorliegenden Erfindung ist die in Schritt a) ausgewählte Silanverbindung ein gemischtes oder ungemischtes Tetraalkoxysilan gemäß der allgemeinen Formel Si(OCₓH₂ₓ₊₁)₄ mit x = 1-12, welches bevorzugt ausgewählt ist aus der Gruppe bestehend aus Tetraethoxysilan, Tetrapropoxysilan, Tetrabutoxysilan und Mischungen hiervon, wobei Tetraethoxysilan besonders bevorzugt ist.

Eine weitere bevorzugte Ausführungsform der vorliegenden Erfindung sieht vor, dass der Alkohol aus Schritt a) ein einwertiger, zweiwertiger oder dreiwertiger, verzweigter oder unverzweigter Alkohol ist, der aliphatisch oder aromatisch sein kann und der bevorzugt ausgewählt ist aus der Gruppe bestehend aus Ethanol, Propanol, Butanol, Ethylenglycol, Phenol und Mischungen hiervon, wobei Ethanol bevorzugt ist.

Gemäß einer anderen bevorzugten Ausführungsform der vorliegenden Erfindung ist die in Schritt a) ausgewählte Silanverbindung ein gemischtes oder ungemischtes Tetraalkoxysilan gemäß der allgemeinen Formel Si(OCₓH₂ₓ₊₁)₄ mit x = 1-12, welches bevorzugt ausgewählt ist aus der Gruppe bestehend aus Tetraethoxysilan, Tetrapropoxysilan, Tetrabutoxysilan und Mischungen hiervon, wobei Tetraethoxysilan besonders bevorzugt ist und der Alkohol aus Schritt a) ein einwertiger, zweiwertiger oder dreiwertiger, verzweigter oder unverzweigter Alkohol ist, der aliphatisch oder aromatisch sein kann und der bevorzugt ausgewählt ist aus der Gruppe bestehend aus Ethanol, Propanol, Butanol, Ethylenglycol, Phenol und Mischungen hiervon, wobei Ethanol bevorzugt ist.

Eine weitere bevorzugte Ausführungsform der vorliegenden Erfindung sieht vor, dass die organische Säure in Schritt b) ausgewählt ist aus der Gruppe bestehend aus Sulfonsäuren, insbesondere Methansulfonsäure, Carbonsäuren, Carbonsäureestern, Schwefelsäureestern, Aminosäuren, Phosphonsäuren, Phosphorsäureestern und Mischungen hiervon, wobei Methansulfonsäure besonders bevorzugt ist.

Gemäß einer anderen bevorzugten Ausführungsform vorliegender Erfindung ist die organische Säure in Schritt b) 0,01 bis 1 N und besonders bevorzugt 0,1 N.

Eine weitere bevorzugte Ausführungsform sieht vor, dass der Gehalt an organischer Säure in der wässrigen Lösung aus Schritt b) im Bereich von 0,01 bis 2 Gew.-%, bevorzugt 0,05 bis 1 Gew.-% und besonders bevorzugt 0,1 bis 0,5 Gew.-%, bezogen auf das Gesamtgewicht der Lösung, liegt.

Nach einer anderen bevorzugten Ausführungsform vorliegender Erfindung ist die organische Säure in Schritt b) ausgewählt aus der Gruppe bestehend aus Sulfonsäuren, insbesondere Methansulfonsäure, Carbonsäuren, Carbonsäureestern, Schwefelsäureestern, Aminosäuren, Phosphonsäuren, Phosphorsäureestern und Mischungen hiervon, wobei Methansulfonsäure besonders bevorzugt ist und die organische Säure in Schritt b) 0,01 bis 1 N und besonders bevorzugt 0,1 N ist.

Gemäß einer anderen bevorzugten Ausführungsform vorliegender Erfindung ist die organische Säure in Schritt b) ausgewählt aus der Gruppe bestehend aus Sulfonsäuren, insbesondere Methansulfonsäure, Carbonsäuren, Carbonsäureestern, Schwefelsäureestern, Aminosäuren, Phosphonsäuren, Phosphorsäureestern und Mischungen hiervon, wobei Methansulfonsäure besonders bevorzugt ist und die organische Säure in Schritt b) 0,01 bis 1 N und besonders bevorzugt 0,1 N ist und der Gehalt an organischer Säure in der wässrigen Lösung aus Schritt b) im Bereich von 0,01 bis 2 Gew.-%, bevorzugt 0,05 bis 1 Gew.-% und besonders bevorzugt 0,1 bis 0,5 Gew.-%, bezogen auf das Gesamtgewicht der Lösung, liegt.

Eine weitere bevorzugte Ausführungsform der vorliegenden Erfindung sieht vor, dass das Vermischen in Schritt c) durch Zutropfen der wässrigen Lösung der organischen Säure zur alkoholischen Silanlösung erfolgt oder das Vermischen in Schritt c) durch Zutropfen der alkoholischen Silanlösung zur wässrigen Lösung der organischen Säure erfolgt oder das Vermischen in Schritt c) durch simultanes Zusammengeben der alkoholischen Silanlösung und der wässrigen Lösung der organischen Säure erfolgt.

Nach einer weiteren bevorzugten Ausführungsform der vorliegenden Erfindung erfolgt das Durchmischen gemäß Schritt c) durch Rühren oder Schütteln.

Gemäß einer weiteren bevorzugten Ausführungsform vorliegender Erfindung wird die Mischung aus Schritt c) für 5 Stunden bis 144 Stunden, bevorzugt 10 bis 24 Stunden und besonders bevorzugt für 16 bis 18 Stunden gerührt wird. Eine andere bevorzugte Ausführungsform vorliegender Erfindung sieht vor, dass die Temperatur in Schritt c) 20 bis 60°C, bevorzugt 20 bis 50°C und besonders bevorzugt 25 bis 40°C beträgt.

Nach einer weiteren bevorzugten Ausführungsform der vorliegenden Erfindung beträgt der pH-Wert der Mischung in Schritt c) < 5, bevorzugt 1 bis 4,9 und besonders bevorzugt 4 bis 4,9.

Eine weitere bevorzugte Ausführungsform der vorliegenden Erfindung sieht vor, dass das Vermischen in Schritt c) durch Zutropfen der wässrigen Lösung der organischen Säure zur alkoholischen Silanlösung erfolgt oder das Vermischen in Schritt c) durch Zutropfen der alkoholischen Silanlösung zur wässrigen Lösung der organischen Säure erfolgt oder das Vermischen in Schritt c) durch simultanes Zusammengeben der alkoholischen Silanlösung und der wässrigen Lösung der organischen Säure erfolgt und das Durchmischen gemäß Schritt c) durch Rühren erfolgt und die Mischung aus Schritt c) für 5 Stunden bis 144 Stunden, bevorzugt 10 bis 24 Stunden und besonders bevorzugt für 16 bis 18 Stunden gerührt wird und die Temperatur in Schritt c) 20 bis 60°C, bevorzugt 20 bis 50°C und besonders bevorzugt 25 bis 40°C beträgt und der pH-Wert der Mischung in Schritt c) < 5, bevorzugt 1 bis 4,9 und besonders bevorzugt 4 bis 4,9 beträgt.

Nach einer anderen bevorzugten Ausführungsform der vorliegenden Erfindung werden während Schritt d) 40 bis 80 Gew.-%, bevorzugt 45 bis 75 Gew.-% und besonders bevorzugt 50 bis 65 Gew.-% des Lösungsmittelgemisches, bezogen auf die in Schritt b) bereitgestellte Gesamtmasse des Ansatzes, entfernt.

Gemäß einer weiteren bevorzugten Ausführungsform vorliegender Erfindung wird die konzentrierte Mischung aus Schritt d) auf eine Temperatur von 20 bis -25°C, bevorzugt 10 bis -25°C und besonders bevorzugt 4 bis -20°C, abgekühlt.

Eine weitere bevorzugte Ausführungsform sieht vor, dass während Schritt d) auch zumindest ein Teil des Wassers aus Schritt b) entfernt wird.

Nach einer weiteren bevorzugten Ausführungsform vorliegender Erfindung werden während Schritt d) 40 bis 80 Gew.-%, bevorzugt 45 bis 75 Gew.-% und besonders bevorzugt 50 bis 65 Gew.-% des Lösungsmittelgemisches, bezogen auf die in Schritt b) bereitgestellte Gesamtmasse des Ansatzes, entfernt und die konzentrierte Mischung aus Schritt d) wird auf eine Temperatur von 20 bis -25°C, bevorzugt 10 bis -25°C und besonders bevorzugt 4 bis -20°C, abgekühlt und zumindest ein Teil des Wassers aus Schritt b) wird entfernt.

Nach einer weiteren bevorzugten Ausführungsform vorliegender Erfindung wird Schritt e) ausgeführt bis eine Viskosität von 10 bis 70 Pa·s, bevorzugt 10 bis 40 Pa·s und besonders bevorzugt 20 bis 25 Pa·s erreicht ist.

Eine andere bevorzugte Ausführungsform vorliegender Erfindung sieht vor, dass das Verspinnen unter Anlegen von Druck, im Bereich von 10 bis 60 bar, bevorzugt 10 bis 40 bar und besonders bevorzugt 20 bis 40 bar, erfolgt.

Eine weitere bevorzugte Ausführungsform vorliegender Erfindung sieht vor, dass das Verfahren die folgenden weiteren Verfahrensschritte enthält, die nach Schritt f) erfolgen:
g) Schneiden oder Stanzen der nach dem Verspinnen in Schritt f) erhaltenen Endlosfasern, und/oder
h) Sterilisieren der Fasern aus Schritt f) oder g), bevorzugt durch γ-Strahlung oder durch Behandlung mit Ehylenoxid, einer 70 %-igen Ethanollösung oder Chloroform.

Gemäß einer anderen bevorzugten Ausführungsform der vorliegenden Erfindung wird in Schritt a) genau ein Alkohol als Lösungsmittel verwendet.

Eine weitere bevorzugte Ausführungsform der vorliegenden Erfindung sieht vor, dass in der Lösung gemäß Schritt a) genau eine Silanverbindung gelöst ist.

Nach einer weiteren bevorzugten Ausführungsform vorliegender Erfindung wird in Schritt b) eine wässrige Lösung genau einer organischen Säure bereitgestellt.

Eine weitere bevorzugte Ausführungsform vorliegender Erfindung sieht vor, dass in Schritt a) genau ein Alkohol als Lösungsmittel verwendet wird und in der Lösung gemäß Schritt a) genau eine Silanverbindung gelöst ist und in Schritt b) eine wässrige Lösung genau einer organischen Säure bereitgestellt wird.

Nach einer weiteren bevorzugten Ausführungsform der vorliegenden Erfindung wird das Verfahren kontinuierlich oder chargenweise durchgeführt wird.

Gemäß einer anderen bevorzugten Ausführungsform vorliegender Erfindung enthält das Verfahren keine anderen Prozessschritte als die oben genannten Schritte a) bis h).

### Biodegradierbare Faser

Die vorliegende Erfindung betrifft weiterhin eine nach dem erfindungsgemäßen Verfahren herstellbare Faser.

Gemäß einer bevorzugten Ausführungsform der vorliegenden Erfindung degradiert die Faser in einem Zeitraum von > 6 Monaten, wie z.B. mehr als 3 Jahre, besonders bevorzugt von 6 bis 18 Monaten und insbesondere bevorzugt von 10 bis 12 Monaten.

Eine weitere bevorzugte Ausführungsform vorliegender Erfindung sieht vor, dass die Faser aufwickelbar ist.

Nach einer anderen bevorzugten Ausführungsform vorliegender Erfindung beträgt der Anteil an funktionellen Gruppen aus der organischen Säure, die im Netzwerk kovalent angebunden sind 30 bis 80 %, bevorzugt 45 bis 75 % und besonders bevorzugt 53 bis 65 %, bezogen auf die in Schritt b) bereitgestellten funktionellen Gruppen.

### Verwendung

Die vorliegende Erfindung betrifft weiterhin die Verwendung der erfindungsgemäßen Fasern oder von Fasergeflechten aus diesen Fasern.

Bevorzugt ist die Verwendung in den folgenden Bereichen:
- Regenerative Therapien, bevorzugt als Zellträgermaterial für den Aufbau von Geweben oder Zellkulturen oder als Lichtleiter, besonders bevorzugt im Bereich minimal-invasive Chirurgie;
- Mikrobiologie, bevorzugt werden Bakterien, Hefen, Pilze auf den Fasern aufgebracht oder kultiviert;
- Pharmazeutische Anwendungen, bevorzugt zur Verkapselung oder Ankopplung von Wirkstoffen, Enzymen oder Nanopartikeln;
- Kosmetikindustrie, bevorzugt durch Integration der Fasern in kosmetische Produkte;
- Diagnostik, bevorzugt in vitro und/oder in vivo, besonders bevorzugt zur kovalenten oder adsorptiven Anbindung von Antikörpern, Aptameren und/oder Enzym-Substrat-Komplexen;
- Lebensmittelbranche, bevorzugt zur Verbesserung des Mundgefühls, besonders bevorzugt durch Integration von Aromastoffen, Fetten, Eiweißen, Enzymen, Ionen, Lebensmittelzusatzstoffen;
- Filtern, bevorzugt zum Neutralisieren von Abwässern oder zur Abgabe von Ionen an Flüssigkeiten;
- Faserverstärkung von Werkstoffen, bevorzugt in Kompositen;
- Optik, bevorzugt in der Lichttherapie oder Endoskopie.

Anhand der nachfolgenden Beispiele soll der erfindungsgemäße Gegenstand näher erläutert werden, ohne diesen auf die hier gezeigten spezifischen Ausführungsformen einschränken zu wollen.

### Ausführungsbeispiele

### 1) Methansulfonsäure

5 mol Tetraethoxysilan (Sigma Aldrich) wurden in ethanolischer Lösung (400 mL) vermischt. Zu diesem Sol wurden über 2 h hinweg 0,02 mol Methansulfonsäure (Sigma Aldrich) in einer 0,1 N wässrigen Lösung zugetropft und die entstandene Mischung für weitere 18 h bei 40°C gerührt. Hierbei wurde die Methansulfonsäure kovalent in das Si-0-Gerüst eingebaut (Nachweis siehe unten).

Dem Sol wurden anschließend 906,5 g Lösungsmittel entzogen und es wurde bei -20°C bis zu einer honigartigen Viskosität (21 Pa*s gemessen bei 4 °C) gereift. Die viskose Flüssigkeit wurde in einen auf -15°C temperierten Druckbehälter gefüllt und mit einem Druck von 20 bar durch eine Düsenplatte mit sieben Düsen (Düsendurchmesser: 150 µm) gepresst. Nach einer Fallstrecke von 2,5 m werden die Fasern aufgefangen und bei einer Luftfeuchtigkeit von 20 % gelagert. Die im Artikel "Nanostructured surfaces of biodegradable silica fibers enhance direct amoeboid cell migration in a microtubule-dependent process" von Martin Emmert, Patrick Witzel, Miranda Rothenburger-Glaubitt und Doris Heinrich, der in "The Royal Society of Chemistry" 2017, im Volume 7 auf den Seiten 5708 bis 5714 veröffentlicht wurde, angegebenen Spinnbedingungen sind ebenfalls sehr gut zum Verspinnen der erfindungsgemäßen Fasern geeignet.

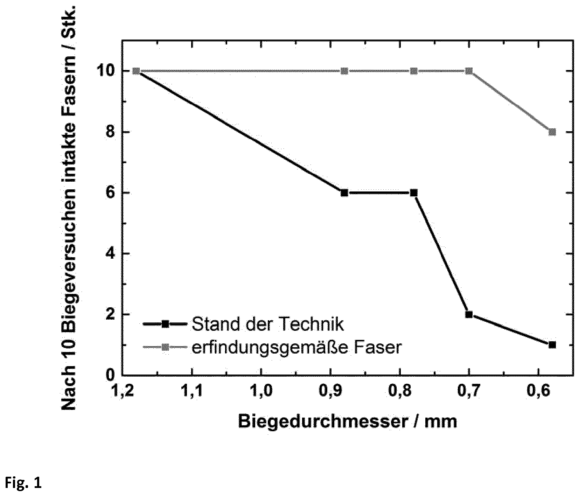

Die Fasern wurden bei niedriger Luftfeuchtigkeit gelagert und wiesen sehr flexible Eigenschaften auf. Zum Test der flexiblen Eigenschaften wurde eine erfindungsgemäße Faser um einen zylindrischen Stab mit verschiedenen Durchmessern gewickelt und geprüft, ob die Faser bei diesem Durchmesser bricht oder intakt bleibt. Dieser Versuch wurde mit insgesamt 10 verschiedenen Fasern durchgeführt und die Anzahl der intakten Fasern nach den 10 Versuchen für jeden Stabdurchmesser aufgetragen. Als Vergleichssystem wurde eine Faser aus dem Stand der Technik verwendet, deren Herstellung erfolgte nach DE 196 09 551 C1. Wie Figur 1 entnommen werden kann weist die erfindungsgemäße Faser (MSS-Faser) flexiblere Eigenschaften auf als die Faser aus dem Stand der Technik (HNO₃-Faser) und bricht erst ab einem Durchmesser von < 0,7 mm.

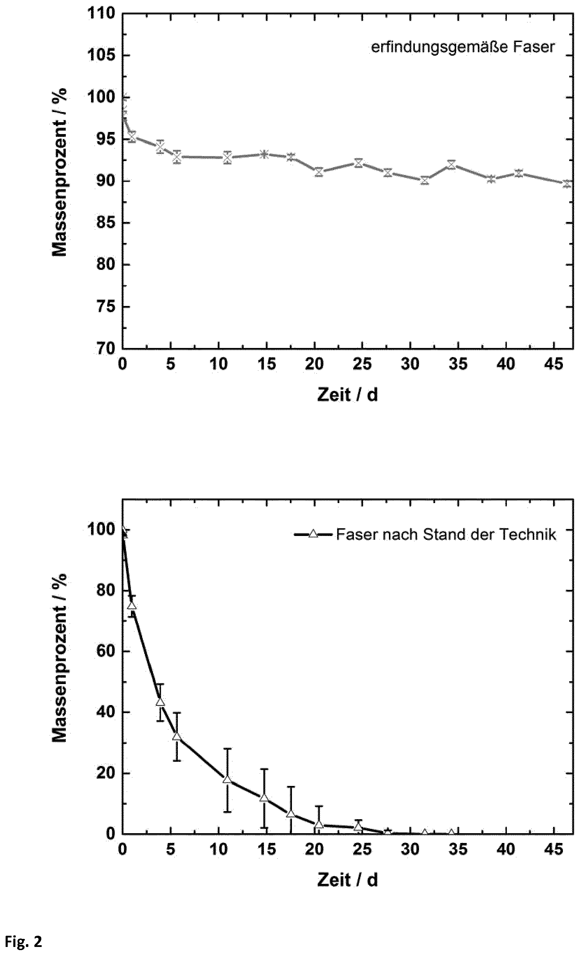

Figur 2 (oben) zeigt das Degradationsprofil einer erfindungsgemäßen Faser in einer phosphatgepufferten Salzlösung (PBS) und der untere Teil von Figur 2 zeigt das Degradationsprofil einer nach dem Stand der Technik hergestellten Faser. Die Versuche wurden statisch durchgeführt, d.h. das Degradationsmedium wurde an jedem Messtag ausgetauscht. Das Degradationsprofil wurde gravimetrisch erstellt, indem die Fasern am jeweiligen Messtag entnommen, zur Gewichtskonstanz getrocknet und die verbleibende Fasermasse ermittelt wurde. Danach wurden die Fasern zur weiteren Degradation wieder ins Degradtionsmedium gegeben.

Den Figuren kann entnommen werden, dass die erfindungsgemäße Faser langsam degradiert, wohingegen die Faser aus dem Stand der Technik bereits innerhalb von 5 Wochen komplett degradiert war.

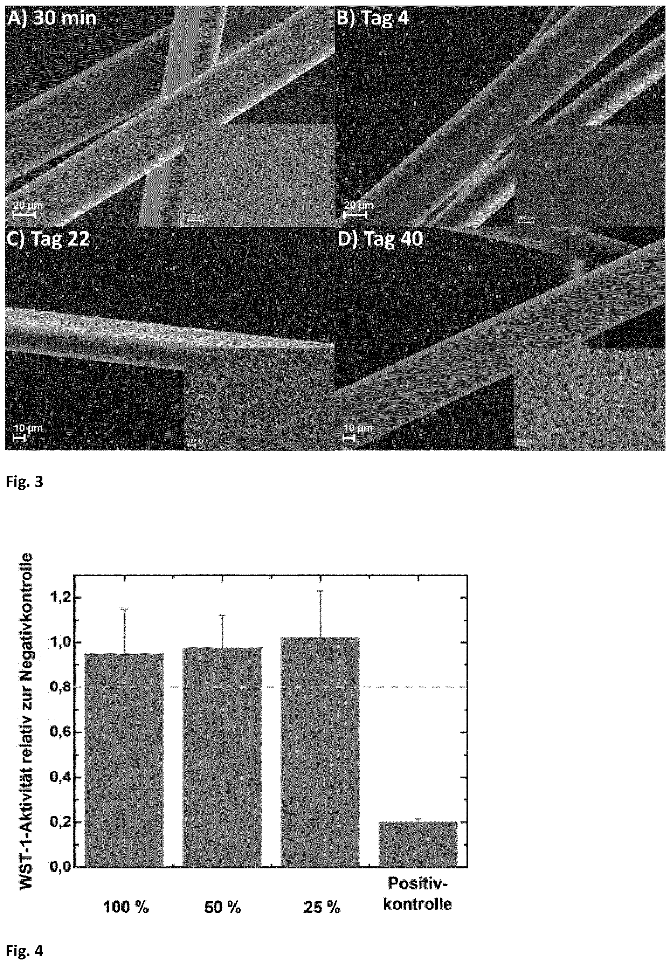

Figur 3 zeigt REM-Aufnahmen der erfindungsgemäßen Faser nach 30 Minuten, 4 Tagen, 22 Tagen und 40 Tagen Lagerung in PBS. Ein Fortschreiten der Degradation ist deutlich zu beobachten.

Weiterhin wurde in Anlehnung an DIN ISO 10993-5 ein Biokompatibilitätstest mit der erfindungsgemäßen Faser durchgeführt. Die Daten sind in Figur 4 zusammengefasst und die erfindungsgemäße Faser ist biokompatibel, Werte über 80 % gelten als nicht zytotoxisch.

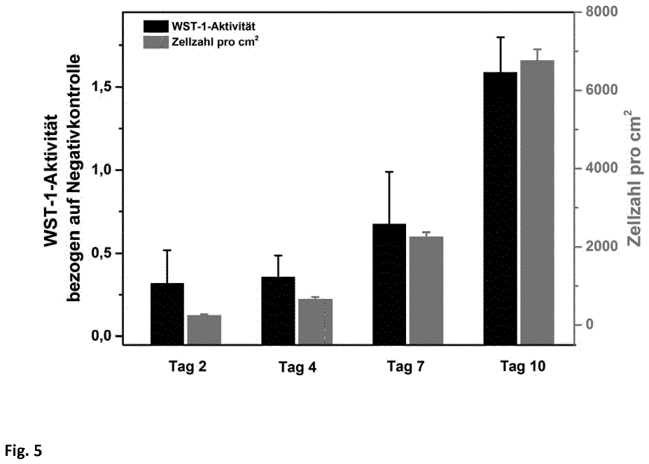

In Figur 5 sind Resultate von Zellproliferationsuntersuchungen mit der erfindungsgemäßen Faser zusammengefasst.

Hierzu wurden 60 mg Fasermaterial mit einer phosphatgepufferten Salzlösung (PBS) gewaschen und in einer 12-Well-Platte mit 50'000 humanen dermalen Fibroblasten (hdF) besiedelt. Die besiedelten Faservliese wurden für 2, 4, 7 und 10 Tage in einem Zellkulturmedium (DMEM) mit 10 % fetalem Kälberserum inkubiert. An jedem Messtag wurden die Fasern in eine neue Wellplatte überführt und die WST-1-Aktivität der Zellen gemessen sowie die Zellzahl bestimmt. Als Negativkontrolle wurden Zellen in einem Well ohne Fasern mit DMEM (+10 % FCS) und als Positivkontrolle mit DMEM (+10 % FCS) mit 1% Natriumdodecylsulfat (SDS) inkubiert.

Der Figur ist zu entnehmen, dass auf der erfindungsgemäßen Faser humane, dermale Fibroblasten sehr schneller proliferieren. Weiterhin zeigt die erfindungsgemäße Faser eine hohe Stoffwechselaktivität der Zellen.

Wie thermogravimetrische Analysen unter synthetischer Luftatmosphäre gekoppelt mit einem Massenspektrometer (TGA-MS) zeigten, wird SOₓ erst bei Temperaturen von über 400°C frei. Dies beweist die kovalente Anbindung der Sulfonsäure.

### 2) Para-Toluolsulfonsäure

5 mol Tetraethoxysilan (Sigma Aldrich) wurden in ethanolischer Lösung (400 mL) vermischt. Zu diesem Sol wurden über 2 h hinweg 0,02 mol para-Toluolsulfonsäure (Sigma Aldrich) in einer 0,1 N wässrigen Lösung zugetropft und die entstandene Mischung für weitere 18 h bei 40°C gerührt. Hierbei wurde die para-Toluolsulfonsäure kovalent in das Si-0-Gerüst eingebaut.

Dem Sol wurden anschließend 906,5 g Lösungsmittel entzogen und es wurde bei -20°C bis zu einer honigartigen Viskosität (22 Pa*s gemessen bei 4 °C) gereift. Die viskose Flüssigkeit wurde in einen auf -15°C temperierten Druckbehälter gefüllt und mit einem Druck von 20 bar durch eine Düsenplatte mit sieben Düsen (Düsendurchmesser: 150 µm) gepresst. Nach einer Fallstrecke von 2,5 m werden die Fasern aufgefangen und bei einer Luftfeuchtigkeit von 20 % gelagert.

## Patentansprüche

1. Verfahren zur Herstellung von biodegradierbaren Fasern, enthaltend die folgenden Schritte:
a) Bereitstellen von zumindest einer alkoholischen Lösung zumindest einer Silanverbindung ausgewählt aus der Gruppe bestehend aus Tetraalkoxysilanen, Trialkoxysilanen, Halogensilanen und Mischungen hiervon;
b) Bereitstellen einer wässrigen Lösung zumindest einer organischen Säure mit einem pKs-Wert von < 2,0, wobei die organische Säure ausgewählt ist aus der Gruppe bestehend aus Sulfonsäuren, Schwefelsäureestern, Phosphonsäuren, Phosphorsäureestern und Mischungen hiervon;
c) Vermischen der in Schritt a) und b) bereitgestellten Lösungen und Durchmischen für 4 Stunden bis 1 Woche bei einer Temperatur von 20 bis 70°C;
d) Entfernen zumindest eines Teils des Alkohols aus der Mischung aus Schritt c) und anschließendes Abkühlen der konzentrierten Mischung auf eine Temperatur von 20 bis -25°C;
e) Aufbewahren der abgekühlten Mischung aus Schritt d) bis eine Viskosität von 10 bis 75 Pa·s, gemessen mit einem Rheometer mit einem koaxialen Zylindermessaufsatz und einer Scherrate von 10 s⁻¹, erreicht ist;
f) Verspinnen der Mischung aus Schritt e) zu Endlosfasern; wobei
während der Schritte c) bis e) eine Vernetzung der Silanverbindung erfolgt und zumindest ein Teil der organischen Säure über kovalente Bindungen in das entstehende Netzwerk eingebaut wird und/oder zur Vernetzung beiträgt.

2. Verfahren nach Anspruch 1, **dadurch gekennzeichnet, dass**
die in Schritt a) ausgewählte Silanverbindung ein gemischtes oder ungemischtes Tetraalkoxysilan gemäß der allgemeinen Formel Si(OCₓH₂ₓ₊₁)₄ mit x = 1-12 ist, welches bevorzugt ausgewählt ist aus der Gruppe bestehend aus Tetraethoxysilan, Tetrapropoxysilan, Tetrabutoxysilan und Mischungen hiervon, wobei Tetraethoxysilan besonders bevorzugt ist; und/oder
der Alkohol aus Schritt a) ein einwertiger, zweiwertiger oder dreiwertiger, verzweigter oder unverzweigter Alkohol ist, der aliphatisch oder aromatisch sein kann und der bevorzugt ausgewählt ist aus der Gruppe bestehend aus Ethanol, Propanol, Butanol, Ethylenglycol, Phenol und Mischungen hiervon, wobei Ethanol bevorzugt ist.

3. Verfahren nach einem der Ansprüche 1 oder 2, **dadurch gekennzeichnet, dass**
die organische Säure in Schritt b) Methansulfonsäure ist; und/oder
die organische Säure in Schritt b) 0,01 bis 1 N und besonders bevorzugt 0,1 N ist; und/oder
der Gehalt an organischer Säure in der wässrigen Lösung aus Schritt b) im Bereich von 0,01 bis 2 Gew.-%, bevorzugt 0,05 bis 1 Gew.-% und besonders bevorzugt 0,1 bis 0,5 Gew.-%, bezogen auf das Gesamtgewicht der Lösung, liegt.

4. Verfahren nach einem der vorhergehenden Ansprüche, **dadurch gekennzeichnet, dass**
das Vermischen in Schritt c) durch Zutropfen der wässrigen Lösung der organischen Säure zur alkoholischen Silanlösung erfolgt; oder
das Vermischen in Schritt c) durch Zutropfen der alkoholischen Silanlösung zur wässrigen Lösung der organischen Säure erfolgt; oder
das Vermischen in Schritt c) durch simultanes Zusammengeben der alkoholischen Silanlösung und der wässrigen Lösung der organischen Säure erfolgt; und/oder
das Durchmischen gemäß Schritt c) durch Rühren oder Schütteln erfolgt; und/oder
die Mischung aus Schritt c) für 5 Stunden bis 144 Stunden, bevorzugt 10 bis 24 Stunden und besonders bevorzugt für 16 bis 18 Stunden gerührt wird; und/oder
die Temperatur in Schritt c) 20 bis 60°C, bevorzugt 20 bis 50°C und besonders bevorzugt 25 bis 40°C beträgt;
der pH-Wert der Mischung in Schritt c) < 5, bevorzugt 1 bis 4,9 und besonders bevorzugt 4 bis 4,9 beträgt.

5. Verfahren nach einem der vorhergehenden Ansprüche, **dadurch gekennzeichnet, dass**
während Schritt d) 40 bis 80 Gew.-%, bevorzugt 45 bis 75 Gew.-% und besonders bevorzugt 50 bis 65 Gew.-% des Lösungsmittelgemisches, bezogen auf die in Schritt b) bereitgestellte Gesamtmasse des Ansatzes, entfernt werden; und/oder
die konzentrierte Mischung aus Schritt d) auf eine Temperatur von 20 bis -25°C, bevorzugt 10 bis -25°C und besonders bevorzugt 4 bis -20°C, abgekühlt wird; und/oder
während Schritt d) auch zumindest ein Teil des Wassers aus Schritt b) entfernt wird.

6. Verfahren nach einem der vorhergehenden Ansprüche, **dadurch gekennzeichnet, dass**
Schritt e) ausgeführt wird bis eine Viskosität von 10 bis 70 Pa·s, bevorzugt 10 bis 40 Pa·s und besonders bevorzugt 20 bis 25 Pa·s erreicht ist, jeweils gemessen mit einem Rheometer mit einem koaxialen Zylindermessaufsatz und einer Scherrate von 10 s⁻¹.

7. Verfahren nach einem der vorhergehenden Ansprüche, **dadurch gekennzeichnet, dass**
das Verspinnen unter Anlegen von Druck, im Bereich von 10 bis 60 bar, bevorzugt 10 bis 40 bar und besonders bevorzugt 20 bis 40 bar, erfolgt.

8. Verfahren nach einem der vorhergehenden Ansprüche, **dadurch gekennzeichnet, dass**
das Verfahren die folgenden weiteren Verfahrensschritte enthält, die nach Schritt f) erfolgen:
g) Schneiden oder Stanzen der nach dem Verspinnen in Schritt f) erhaltenen Endlosfasern, und/oder
h) Sterilisieren der Fasern aus Schritt f) oder g), bevorzugt durch γ-Strahlung oder durch Behandlung mit Ethylenoxid, einer 70 %-igen Ethanollösung oder Chloroform.

9. Verfahren nach einem der vorhergehenden Ansprüche, **dadurch gekennzeichnet, dass**
in Schritt a) genau ein Alkohol als Lösungsmittel verwendet wird und/oder in der Lösung aus Schritt a) genau eine Silanverbindung gelöst ist; und/oder
in Schritt b) eine wässrige Lösung genau einer organischen Säure bereitgestellt wird.

10. Verfahren nach einem der vorhergehenden Ansprüche, **dadurch gekennzeichnet, dass**
das Verfahren kontinuierlich oder chargenweise durchgeführt wird.

11. Biodegradierbare Faser herstellbar nach einem Verfahren gemäß einem der Ansprüche 1 bis 10.

12. Biodegradierbare Faser nach Anspruch 11, **dadurch gekennzeichnet, dass**
die Faser in einem Zeitraum von > 6 Monaten, bevorzugt von 6 bis 18 Monaten und besonders bevorzugt von 10 bis 12 Monaten degradiert, bestimmt mit einem Degradationsprofil, welches gravimetrisch erstellt wird, indem die Fasern am jeweiligen Messtag einem Degradationsmedium entnommen, zur Gewichtskonstanz getrocknet und die verbleibende Fasermasse bestimmt wird, wobei die Fasern danach zur weiteren Degradation wieder ins Degradationsmedium gegeben werden.

13. Biodegradierbare Faser nach einem der Ansprüche 11 oder 12, **dadurch gekennzeichnet, dass**
die Faser aufwickelbar ist und/oder
der Anteil an funktionellen Gruppen aus der organischen Säure, die im Netzwerk kovalent angebunden sind 30 bis 80 %, bevorzugt 45 bis 75 % und besonders bevorzugt 53 bis 65 %, bezogen auf die in Schritt b) bereitgestellten funktionellen Gruppen, beträgt.

14. Fasergeflecht oder Filter enthaltend biodegradierbare Fasern gemäß einem der Ansprüche 11 bis 13 oder bestehend aus diesen Fasern.

15. Verwendung von biodegradierbaren Fasern gemäß einem der Ansprüche 11 bis 13 oder von Fasergeflechten gemäß Anspruch 14 in den Bereichen
• Regenerative Therapien, bevorzugt als Zellträgermaterial für den Aufbau von Geweben oder Zellkulturen oder als Lichtleiter, besonders bevorzugt im Bereich minimal-invasive Chirurgie;
• Mikrobiologie, bevorzugt werden Bakterien, Hefen, Pilze auf den Fasern aufgebracht oder kultiviert;
• Pharmazeutische Anwendungen, bevorzugt zur Verkapselung oder Ankopplung von Wirkstoffen, Enzymen oder Nanopartikeln;
• Kosmetikindustrie, bevorzugt durch Integration der Fasern in kosmetische Produkte;
• Diagnostik, bevorzugt in vitro und/oder in vivo, besonders bevorzugt zur kovalenten oder adsorptiven Anbindung von Antikörpern, Aptameren und/oder Enzym-Substrat-Komplexen;
• Lebensmittelbranche, bevorzugt zur Verbesserung des Mundgefühls, besonders bevorzugt durch Integration von Aromastoffen, Fetten, Eiweißen, Enzymen, Ionen, Lebensmittelzusatzstoffen;
• Filtern, bevorzugt zum Neutralisieren von Abwässern oder zur Abgabe von Ionen an Flüssigkeiten;
• Faserverstärkung von Werkstoffen, bevorzugt in Kompositen;
• Optik, bevorzugt in der Lichttherapie oder Endoskopie.

## Claims

1. Process for manufacturing biodegradable fibers including the following steps:
a) Providing at least one alcoholic solution of at least one silane compound selected from the group consisting of tetraalkoxysilanes, trialkoxysilanes, halosilanes, and mixtures thereof;
b) Providing an aqueous solution of at least one organic acid having a pKs value of < 2.0, wherein the organic acid is selected from the group consisting of sulfonic acids, sulfuric acid esters, phosphonic acids, phosphoric acid esters and mixtures thereof;
c) Mixing the solutions provided in steps a) and b) and mixing for 4 hours to 1 week at a temperature of 20 to 70°C;
d) Removing at least part of the alcohol from the mixture from step c) and then cooling the concentrated mixture to a temperature of 20 to -25°C;
e) Keeping the cooled mixture from step d) until a viscosity of 10 to 75 Pa s, measured with a rheometer with a coaxial cylinder measuring attachment and a shear rate of 10 s⁻¹, is reached;
f) Spinning the mixture from step e) into continuous fibers;
wherein
during steps c) to e), crosslinking of the silane compound takes place and at least part of the organic acid is incorporated into the resulting network via covalent bonds and/or contributes to the crosslinking.

2. Process according to claim 1, **characterized in that**
the silane compound selected in step a) is a mixed or unmixed tetraalkoxysilane according to the general formula Si(OCₓH₂ₓ₊₁)₄ where x = 1-12, which is preferably selected from the group consisting of tetraethoxysilane, tetrapropoxysilane, tetra-butoxysilane and mixtures thereof, tetraethoxysilane being particularly preferred; and/or
the alcohol of step a) is a monovalent, divalent or trivalent, branched or unbranched alcohol which may be aliphatic or aromatic and which is preferably selected from the group consisting of ethanol, propanol, butanol, ethylene glycol, phenol and mixtures thereof, ethanol being preferred.

3. Process according to any one of claims 1 or 2, **characterized in that**
the organic acid in step b) is methanesulfonic acid; and/or
the organic acid in step b) is 0.01 to 1 N and particularly preferably 0.1 N; and/or
the content of organic acid in the aqueous solution from step b) is in the range from 0.01 to 2 % by weight, preferably from 0.05 to 1 % by weight and particularly preferably from 0.1 to 0.5 % by weight, based on the total weight of the solution.

4. Process according to any one of the preceding claims, **characterized in that**
the mixing in step c) is carried out by dripping the aqueous solution of the organic acid into the alcoholic silane solution; or
the mixing in step c) is carried out by dripping the alcoholic silane solution to the aqueous solution of the organic acid; or
the mixing in step c) is carried out by simultaneously combining the alcoholic silane solution and the aqueous solution of the organic acid; and/or
the mixing according to step c) is carried out by stirring or shaking; and/or
the mixture from step c) is stirred for 5 hours to 144 hours, preferably 10 to 24 hours and particularly preferably for 16 to 18 hours; and/or
the temperature in step c) is 20 to 60°C, preferably 20 to 50°C and particularly preferably 25 to 40°C;
the pH value of the mixture in step c) is < 5, preferably 1 to 4.9 and particularly preferably 4 to 4.9.

5. Process according to any one of the preceding claims, **characterized in that**
during step d) 40 to 80 % by weight, preferably 45 to 75 % by weight and particularly preferably 50 to 65 % by weight of the solvent mixture, based on the total mass of the preparation prepared in step b), are removed; and/or
the concentrated mixture from step d) is cooled to a temperature of 20 to -25°C, preferably 10 to -25°C and particularly preferably 4 to -20°C; and/or
during step d) at least part of the water from step b) is also removed.

6. Process according to any one of the preceding claims, **characterized in that**
step e) is carried out until a viscosity of 10 to 70 Pa s, preferably 10 to 40 Pa s and particularly preferably 20 to 25 Pa s is achieved, in each case measured using a rheometer with a coaxial cylinder measuring attachment and a shear rate of 10 s⁻¹.

7. Process according to any one of the preceding claims, **characterized in that**
the spinning is carried out under pressure in the range from 10 to 60 bar, preferably 10 to 40 bar and particularly preferably 20 to 40 bar.

8. Process according to any one of the preceding claims, **characterized in that**
the process includes the following further process steps, which are carried out after step f):
g) Cutting or punching the continuous fibers obtained after the spinning in step f), and/or
h) Sterilizing the fibres from step f) or g), preferably by γ-radiation or by treatment with ethylene oxide, a 70 % ethanol solution or chloroform.

9. Process according to any one of the preceding claims, **characterized in that**
exactly one alcohol is used as solvent in step a) and/or exactly one silane compound is dissolved in the solution from step a); and/or
in step b) an aqueous solution of exactly one organic acid is provided.

10. Process according to any one of the preceding claims, **characterized in that**
the process is performed continuously or in batches.

11. Biodegradable fiber, which can be manufactured by a process according to any one of claims 1 to 10.

12. Biodegradable fiber according to claim 11, **characterized in that**
the fiber degrades over a time period of > 6 months, preferably from 6 to 18 months and particularly preferably from 10 to 12 months, determined using a degradation profile which is created gravimetrically by removing the fibers from a degradation medium on the respective measurement day, drying them to constant weight and determining the remaining fiber mass, the fibers then being returned to the degradation medium for further degradation.

13. Biodegradable fiber according to claim 11 or 12, **characterized in that**
the fiber can be wound up and/or
the proportion of functional groups from the organic acid which are covalently bonded in the network is 30 to 80 %, preferably 45 to 75 % and particularly preferably 53 to 65 %, based on the functional groups provided in step b).

14. Fiber mesh or filter including biodegradable fibers according to one of claims 11 to 13 or consisting of these fibers.

15. Use of biodegradable fibers according to any one of claims 11 to 13 or of fiber mesh according to claim 14 in the areas of
• regenerative therapies, preferably as a cell carrier material for the composition of tissues or cell cultures or as a light guide, particularly preferred in the field of minimally invasive surgery;
• microbiology, preferably bacteria, yeasts, fungi are applied to the fibers or cultivated;
• pharmaceutical applications, preferably for the encapsulation or coupling of active ingredients, enzymes or nanoparticles;
• cosmetics industry, preferably by integrating the fibers into cosmetic products;
• diagnostics, preferably in vitro and/or in vivo, particularly preferably for covalent or adsorptive binding of antibodies, ap-tamers and/or enzyme-substrate complexes;
• food industry, preferably to improve mouthfeel, particularly preferably by integrating flavorings, fats, proteins, enzymes, ions, food additives;
• filters, preferably for neutralizing waste water or for releasing ions into liquids;
• fiber reinforcement of materials, preferably in composites;
• optics, preferably in light therapy or endoscopy.

## Revendications

1. Procédé de production de fibres biodégradables, comprenant les étapes suivantes :
a) la fourniture d'au moins une solution alcoolique d'au moins un composé silane choisi dans le groupe constitué par les tétraal-coxysilanes, les trialcoxysilanes, les halogénosilanes et leurs mélanges ;
b) la fourniture d'une solution aqueuse d'au moins un acide organique avec un pKs < 2,0, dans lequel l'acide organique est choisi dans le groupe constitué par les acides sulfoniques, les esters d'acide sulfurique, les acides phosphoniques, les esters d'acide phosphorique et leurs mélanges ;
c) le mélange des solutions fournies dans les étapes a) et b) et l'agitation pendant 4 heures à 1 semaine à une température de 20 à 70 °C ;
d) l'élimination d'au moins une partie de l'alcool du mélange de l'étape c), puis le refroidissement du mélange concentré à une température de 20 à -25 °C ;
e) la conservation du mélange refroidi de l'étape d) jusqu'à ce qu'une viscosité de 10 à 75 Pa·s, mesurée avec un rhéomètre avec un accessoire de mesure cylindrique coaxial et un taux de cisaillement de 10 s⁻¹, soit atteinte ;
f) le filage du mélange de l'étape e) en fibres continues ; dans lequel
pendant les étapes c) à e), il se produit une réticulation du composé silane et au moins une partie de l'acide organique est incorporée dans le réseau formé par l'intermédiaire de liaisons covalentes et/ou contribue à la réticulation.

2. Procédé selon la revendication 1, **caractérisé en ce que**
le composé silane choisi dans l'étape a) est un tétraalcoxysilane mélangé ou non mélangé selon la formule générale Si(OCₓH₂ₓ₊₁)₄ avec x = 1-12, lequel est de préférence choisi dans le groupe constitué par le tétraéthoxysilane, le tétrapropoxysilane, le tétrabutoxysilane et leurs mélanges, dans lequel le tétraéthoxysilane est particulièrement préféré ; et/ou
l'alcool de l'étape a) est un alcool monovalent, divalent ou trivalent, ramifié ou non ramifié, qui peut être aliphatique ou aromatique et qui est de préférence choisi dans le groupe constitué par l'éthanol, le propanol, le butanol, l'éthylèneglycol, le phénol et leurs mélanges, dans lequel l'éthanol est préféré.

3. Procédé selon l'une quelconque des revendications 1 ou 2, **caractérisé en ce que**
l'acide organique dans l'étape b) est l'acide méthanesulfo-nique ; et/ou
l'acide organique dans l'étape b) est de 0,01 à 1 N et de manière particulièrement préférée 0,1 N ; et/ou
la teneur en acide organique dans la solution aqueuse de l'étape b) se situe dans la plage de 0,01 à 2 % en poids, de préférence de 0,05 à 1 % en poids et de manière particulièrement préférée de 0,1 à 0,5 % en poids, par rapport au poids total de la solution.

4. Procédé selon l'une quelconque des revendications précédentes, **caractérisé en ce que**
le mélange dans l'étape c) s'effectue par ajout goutte à goutte de la solution aqueuse de l'acide organique à la solution alcoolique de silane ; ou
le mélange dans l'étape c) s'effectue par ajout goutte à goutte de la solution alcoolique de silane à la solution aqueuse de l'acide organique ; ou
le mélange dans l'étape c) s'effectue par coalescence simultanée de la solution alcoolique de silane et de la solution aqueuse de l'acide organique ; et/ou
le mélange intime selon l'étape c) s'effectue par agitation ou secouage ; et/ou
le mélange de l'étape c) est agité pendant 5 heures à 144 heures, de préférence 10 à 24 heures et de manière particulièrement préférée pendant 16 à 18 heures ; et/ou
la température dans l'étape c) est de 20 à 60 °C, de préférence de 20 à 50 °C et de manière particulièrement préférée de 25 à 40 °C ;
le pH du mélange dans l'étape c) est < 5, de préférence de 1 à 4,9 et de manière particulièrement préférée de 4 à 4,9.

5. Procédé selon l'une quelconque des revendications précédentes, **caractérisé en ce que**
pendant l'étape d), 40 à 80 % en poids, de préférence 45 à 75 % en poids et de manière particulièrement préférée 50 à 65 % en poids du mélange de solvants, par rapport à la masse totale de la préparation fournie dans l'étape b) sont éliminés ; et/ou
le mélange concentré de l'étape d) est refroidi à une température de 20 à -25 °C, de préférence de 10 à -25 °C et de manière particulièrement préférée de 4 à -20 °C ; et/ou
pendant l'étape d), au moins une partie de l'eau de l'étape b) est également éliminée.

6. Procédé selon l'une quelconque des revendications précédentes, **caractérisé en ce que**
l'étape e) est réalisée jusqu'à ce qu'une viscosité de 10 à 70 Pa·s, de préférence de 10 à 40 Pa·s et de manière particulièrement préférée de 20 à 25 Pa·s soit atteinte, respectivement mesurée avec un rhéomètre avec un accessoire de mesure cylindrique coaxial et un taux de cisaillement de 10 s⁻¹.

7. Procédé selon l'une quelconque des revendications précédentes, **caractérisé en ce que**
le filage s'effectue avec application d'une pression, dans la plage de 10 à 60 bars, de préférence 10 à 40 bars et de manière particulièrement préférée 20 à 40 bars.

8. Procédé selon l'une quelconque des revendications précédentes, **caractérisé en ce que**
le procédé comprend les étapes de procédé supplémentaires suivantes, qui s'effectuent après l'étape f) :
g) la coupe ou l'estampage des fibres continues obtenues après le filage dans l'étape f), et/ou
h) la stérilisation des fibres de l'étape f) ou g), de préférence par rayonnement γ ou par traitement avec de l'oxyde d'éthylène, une solution d'éthanol à 70 % ou du chloroforme.

9. Procédé selon l'une quelconque des revendications précédentes, **caractérisé en ce que**
dans l'étape a), exactement un alcool est utilisé comme solvant et/ou exactement un composé silane est dissout dans la solution de l'étape a) ; et/ou
dans l'étape b), on prépare une solution aqueuse d'exactement un acide organique.

10. Procédé selon l'une quelconque des revendications précédentes, **caractérisé en ce que**
le procédé est mis en oeuvre en continu ou par lots.

11. Fibre biodégradable pouvant être produite suivant un procédé selon l'une quelconque des revendications 1 à 10.

12. Fibre biodégradable selon la revendication 11, **caractérisée en ce que**
la fibre se dégrade dans un laps de temps > 6 mois, de préférence de 6 à 18 mois et de manière particulièrement préférée de 10 à 12 mois, déterminé avec un profil de dégradation, lequel est établi par gravimétrie, du fait que les fibres sont prélevées le jour de mesure respectif d'un milieu de dégradation, séchées jusqu'à un poids constant et la masse de fibres restante est déterminée, dans laquelle les fibres sont ensuite réintroduites dans le milieu de dégradation pour poursuivre la dégradation.

13. Fibre biodégradable selon l'une quelconque des revendications 11 ou 12, **caractérisée en ce que**
la fibre peut être enroulée et/ou
la proportion de groupes fonctionnels provenant de l'acide organique, qui sont liés de manière covalente dans le réseau, est de 30 à 80 %, de préférence 45 à 75 % et de manière particulièrement préférée 53 à 65 %, par rapport aux groupes fonctionnels fournis dans l'étape b).

14. Tresse fibreuse ou filtre comprenant des fibres biodégradables selon l'une quelconque des revendications 11 à 13 ou constitué(e) de ces fibres.

15. Utilisation de fibres biodégradables selon l'une quelconque des revendications 11 à 13 ou de tresses de fibres selon la revendication 14 dans les domaines
• des thérapies régénératives, de préférence comme matériau de support cellulaire pour la constitution de tissus ou de cultures cellulaires ou comme guide de lumière, de manière particulièrement préférée dans le domaine de la chirurgie mini-invasive ;
• de la microbiologie, de préférence des bactéries, des levures, des champignons sont appliqués ou cultivés sur les fibres ;
• des applications pharmaceutiques, de préférence pour l'encapsulation ou le couplage de substances actives, d'enzymes ou de nanoparticules ;
• de l'industrie cosmétique, de préférence par intégration des fibres dans les produits cosmétiques ;
• du diagnostic, de préférence in vitro et/ou in vivo, de manière particulièrement préférée pour la liaison covalente ou adsorptive d'anticorps, d'aptamères et/ou de complexes enzyme-substrat ;
• du secteur alimentaire, de préférence pour améliorer la sensation en bouche, de manière particulièrement préférée par l'intégration de substances aromatiques, de graisses, de protéines, d'enzymes, d'ions, d'additifs alimentaires ;
• de filtres, de préférence pour neutraliser les eaux usées ou pour fournir des ions aux liquides ;
• de renforcement de matériaux par des fibres, de préférence dans des composites ;
• de l'optique, de préférence en luminothérapie ou en endoscopie.
